# EUROPEAN PATENT SPECIFICATION

(11) **EP 3 628 041 B1**
(45) Date of publication and mention of the grant of the patent: **17.03.2021**
(21) Application number: 18722605.5
(22) Date of filing: 15.05.2018
(51) Int. Cl.: C07C 2/84, C07C 11/04, C07C 5/42

(54) **OXIDATIVE COUPLING OF METHANE**
OXIDATIVE KUPPLUNG VON METHAN
COUPLAGE OXYDATIF DE MÉTHANE

(30) Priority: 16.05.2017 EP 17386019
(43) Date of publication of application: 01.04.2020
(73) Proprietor: Shell Internationale Research Maatschappij B.V., 2596 HR The Hague (NL)
(72) Inventor: MITKIDIS, Georgios, 1031 HW Amsterdam (NL); SAN ROMAN MACIA, Maria, 3747 Ras Laffan (QA)
(74) Representative: Shell Legal Services IP
(86) International application number: PCT/EP2018/062437
(87) International publication number: WO 2018/210782

(56) References cited:
- EP-A1- 1 632 467
- WO-A1-02/24614
- WO-A1-2017/065947
- RU-C1- 2 528 829
- US-A1- 2017 066 700

## Description

### Field of the invention

The present invention relates to a process for oxidative coupling of methane (OCM).

### Background of the invention

Methane is a valuable resource which is used not only as a fuel, but is also used in the synthesis of chemical compounds such as higher hydrocarbons.

The conversion of methane to other chemical compounds can take place via indirect conversion wherein methane is reformed to synthesis gas (hydrogen and carbon monoxide), followed by reaction of the synthesis gas in a Fischer-Tropsch process. However, such indirect conversion is costly and consumes a lot of energy.

Consequently, it is desirable for industry to be able to convert methane directly to other chemical compounds without requiring the formation of intermediates such as synthesis gas. To this end, there has been increasing focus in recent years on the development of processes for the oxidative coupling of methane (OCM).

The oxidative coupling of methane converts methane into saturated and unsaturated, non-aromatic hydrocarbons having 2 or more carbon atoms, including ethylene. In this process, a gas stream comprising methane is contacted with an OCM catalyst and with an oxidant, such as oxygen. In such a process, the oxygen is adsorbed on the catalyst's surface. Methane molecules are then converted into methyl radicals. Two methyl radicals are first coupled into one ethane molecule, which is then dehydrogenated into ethylene via an ethyl radical intermediate.

In general, the conversion that can be achieved in an OCM process is relatively low. Besides, at a higher conversion, the selectivity decreases so that it is generally desired to keep the conversion low. As a result, a relatively large amount of unconverted methane leaves the OCM reactor. The proportion of unconverted methane in the OCM product gas stream may be as high as 60 to 80 mole% based on the total molar amount of the gas stream. Typically, an OCM reactor effluent comprises ethylene, ethane, methane, carbon dioxide and water. The unconverted methane has to be recovered from such effluent and subsequently recycled to the OCM process WO2017/065947 discloses the integration of an OCM process with an ethylene cracker.

It is known to separate the gas stream leaving an OCM process in the following way. Acid gas (mainly CO₂) is removed in two stages, the first stage is an aqueous amine absorption system, using for example monoethanolamine (MEA), and the second stage removes final traces of CO₂ by scrubbing against aqueous NaOH. The CO₂-free gas is dried in a dessicant bed and processed in a separation train similar to that used in conventional ethylene plants. The separation sequence comprises a front end demethanizer, deethanizer, C2 splitter, depropanizer, C3 splitter, and a debutanizer. Methane may be separated by means of cryogenic distillation in so-called "demethanizer" columns. The use of cryogenic distillation following an OCM process is for example disclosed in US5113032 and US5025108.

As mentioned above, the gas stream resulting from an OCM process comprises ethylene, ethane and (unconverted) methane. In a case where ethylene is the desired product, apart from recycling said unconverted methane to the step wherein OCM is effected, it may also be desired to recycle ethane to the OCM step in order to maximise the production of ethylene, as ethane is an intermediate in the production of ethylene vian OCM. However, disadvantageously, after having been recycled to an OCM step, wherein an OCM catalyst is used, ethane tends to be combusted into carbon dioxide rather than dehydrogenated into ethylene.

Therefore, it is an objective of the present invention to provide an improved process for oxidative coupling of methane, wherein the production of ethylene may be maximised, that is to say wherein an increased ethylene selectivity and/or yield may be achieved, and which does not have the above-mentioned and below-mentioned disadvantages. Another objective is to reduce the amount of energy required for such process wherein the production of ethylene may be maximised.

### Summary of the invention

Surprisingly it was found that the above-mentioned objective may be obtained by means of the OCM process of the present invention.

The present invention relates to a process for oxidative coupling of methane (OCM), comprising the steps of:
(a) contacting, in a reactor, oxygen and methane with an OCM catalyst, resulting in a reactor effluent comprising ethylene, ethane, methane, carbon dioxide and water;
(b) cooling at least a part of the reactor effluent as obtained in step (a) to obtain a liquid stream comprising water and a gas stream comprising ethylene, ethane, methane and carbon dioxide;
(c) removing carbon dioxide from at least a part of the gas stream comprising ethylene, ethane, methane and carbon dioxide as obtained in step (b) resulting in a gas stream comprising ethylene, ethane and methane;
(d) recovering a stream comprising methane, a stream comprising ethane and a stream comprising ethylene from at least a part of the gas stream comprising ethylene, ethane and methane as obtained in step (c);
(e) recycling at least a part of the stream comprising methane as obtained in step (d) to step (a);
(f) converting ethane from the stream comprising ethane as obtained in step (d) to ethylene by subjecting the ethane to oxidative dehydrogenation (ODH) conditions.

### Brief description of the drawings

Figure 1 depicts an embodiment of the present invention wherein an effluent from a water condensation unit of an oxidative dehydrogenation (ODH) configuration, said effluent comprising unconverted ethane and ethylene, is fed to an oxidative coupling (OCM) configuration.
Figure 2 depicts an embodiment of the present invention wherein an effluent from a carbon dioxide removal unit of an ODH configuration, said effluent comprising unconverted ethane and ethylene, is fed to an OCM configuration.
Figure 3 depicts an embodiment of the present invention wherein an effluent from a drying unit of an ODH configuration, said effluent comprising unconverted ethane and ethylene, is fed to an OCM configuration.

### Detailed description of the invention

In the process of the present invention, as described above, it has appeared that by converting ethane from a recovered stream comprising ethane to ethylene, by subjecting the ethane to oxidative dehydrogenation (ODH) conditions in step (f) of the present process, the overall production of ethylene from methane in an oxidative coupling of methane (OCM) process may be maximised. The combination of said ODH step (f) with step (e) of the present process, wherein unconverted methane is recycled to OCM step (a), enables a maximisation of the production of ethylene. In such an integrated process, comprising an OCM step as the main ethylene production step and an ODH step as an additional ethylene production step, the overall ethylene selectivity and/or yield may be advantageously increased.

Besides, converting ethane to ethylene in such additional ODH step rather than recycling said ethane to the OCM step, wherein an OCM catalyst is used, prevents the ethane from being combusted in the OCM step. For, as mentioned above, after having been recycled to an OCM step ethane tends to be combusted into carbon dioxide rather than dehydrogenated into ethylene. Such combustion of recycled ethane in an OCM step using an OCM catalyst tends to occur more often as compared to combustion of ethane in an ODH step using an ODH catalyst. Therefore, this also advantageously contributes to the potential for a maximisation of the production of ethylene in the present integrated process.

Furthermore, as an alternative to converting ethane in ODH step (f) of the present integrated process to ethylene, ethane could be converted to ethylene in other ways which do not involve ODH or OCM. For example, ethylene may be produced from ethane by steam cracking (pyrolysis) an ethane stream under the influence of heat, in an oxygen-depleted atmosphere, into a product stream comprising ethylene and hydrogen. However, as compared to ODH step (f) of the present integrated process, such alternative ethane cracking step is disadvantageous in that ethane cracking has a low ethylene selectivity, high energy consumption, big carbon dioxide (CO₂) footprint and high capital intensity. Therefore, in the present process, advantageously use is made of ODH of ethane, which has a high ethylene selectivity, low energy consumption, small CO₂ footprint and low capital intensity.

Both OCM and ethane ODH are exothermic chemical processes, whereas ethane cracking is an endothermic process. This means that the overall energy consumption for producing ethylene and working-up the product streams in the present integrated process is relatively low. In fact, the heat released in the OCM and ODH steps of the present integrated process may advantageously be used elsewhere in said process, for example in the work-up section. Thus, this advantageously results in a higher energy efficiency for the present integrated process. Likewise, the CO₂ footprint of the present integrated process can be kept relatively small.

Further, both OCM step (a) and ODH step (f) require an oxygen (O₂) feed. Therefore, advantageously, a further synergy may be achieved by sharing a common O₂ source for both said ethylene production steps (a) and (f).

Still further, apart from the relatively high amount of methane in an OCM effluent, the product compositions of OCM and ODH effluents are quite similar in that they both comprise ethylene, ethane, carbon dioxide and water. On the contrary, an ethane cracker effluent comprises ethylene, unconverted ethane, hydrogen and generally a relatively high amount of hydrocarbons having 3 or more carbon atoms. Accordingly, as further described below, in the present process the OCM work-up section may advantageously also be used to separate the components from the ODH effluent that results from ODH step (f). Additionally, the following further integration options may advantageously be implemented in the present integrated process, involving the sharing of utilities, for example: 1) to use the same steam system facility for generating steam from the exothermic heat produced in the OCM and ODH reactions; 2) to use the same compressor(s) for pressurizing similar streams generated in the OCM and ODH configurations; 3) to use the same quencher for removing water as produced in the OCM and ODH reactions; and 4) to use the same oxygen generation unit as, as described above, a common O₂ source may be shared.

These and other features and synergies of the present integrated process result in that the production of ethylene may be maximised, and further in that the amount of energy required for such process may be relatively low.

The process of the present invention comprises several steps as further described below. Said process may comprise one or more intermediate steps between these described steps. Further, said process may comprise one or more additional steps preceding a described first step and/or following a described last step.

While the process of the present invention and the stream or streams used in said process are described in terms of "comprising", "containing" or "including" one or more various described steps or components, they can also "consist essentially of" or "consist of" said one or more various described steps or components.

In the context of the present invention, in a case where a stream comprises two or more components, these components are to be selected in an overall amount not to exceed 100 vol.% or 100 wt.%.

### Step (a)

In step (a) of the present integrated process, oxygen and methane are contacted, in a reactor, with an OCM catalyst, resulting in a reactor effluent comprising ethylene, ethane, methane, carbon dioxide and water.

In step (a), the reactor may be any reactor suitable for the oxidative coupling of methane, such as a fixed bed reactor with axial or radial flow and with inter-stage cooling or a fluidized bed reactor equipped with internal and external heat exchangers.

In one embodiment of the present invention, a catalyst composition comprising a methane oxidative coupling (OCM) catalyst may be packed along with an inert packing material, such as quartz, into a fixed bed reactor having an appropriate inner diameter and length.

Optionally, such catalyst composition may be pretreated at high temperature to remove moisture and impurities therefrom. Said pretreatment may take place, for example, at a temperature in the range of from 100-300 °C for about one hour in the presence of an inert gas such as helium.

Various processes and reactor set-ups are described in the OCM field and the process of the present invention is not limited in that regard. The person skilled in the art may conveniently employ any of such processes in the reaction step of the process of the present invention.

Suitable processes include those described in EP0206042A1, US4443649, CA2016675, US6596912, US20130023709, WO2008134484 and WO2013106771.

As used herein, the term "reactor feed" is understood to refer to the totality of the gas stream(s) at the inlet(s) of the reactor. Thus, as will be appreciated by one skilled in the art, the reactor feed is often comprised of a combination of one or more gas stream(s), such as a methane stream, an oxygen stream, an air stream, a recycle gas stream, etc. For example, in one embodiment, a gas stream comprising methane and another gas stream comprising oxygen are fed to the reactor. In another embodiment, a gas stream comprising methane and oxygen is fed to the reactor.

The one gas stream or multiple gas streams which may be fed to the OCM reactor may additionally comprise an inert gas. An inert gas is defined as a gas that does not take part in the oxidative coupling of methane. The inert gas may be selected from the group consisting of the noble gases and nitrogen (N₂). Preferably, the inert gas is nitrogen or argon, more preferably nitrogen. In case air is fed to the reactor, one or multiple gas streams comprise oxygen as well as nitrogen.

During the oxidative coupling of methane in step (a), a reactor feed comprising methane and oxygen may be introduced into the reactor, so that methane and oxygen are contacted with a methane oxidative coupling catalyst inside that reactor.

A gas stream comprising oxygen (to be combined with the methane in step (a)) may be a high purity oxygen stream. Such high-purity oxygen may have a purity greater than 90%, preferably greater than 95%, more preferably greater than 99%, and most preferably greater than 99.4%.

In step (a) of the process of the present invention, methane and oxygen may be added to the reactor as mixed feed, optionally comprising further components therein, at the same reactor inlet. Alternatively, the methane and oxygen may be added in separate feeds, optionally comprising further components therein, to the reactor at the same reactor inlet or at separate reactor inlets. In yet another alternative, oxygen may be provided through a metal oxide, preferably mixed metal oxide, catalyst, which catalyst acts as both a source of oxygen as well as the catalyst for the oxidative coupling of methane reaction. The metal oxide catalyst is introduced to OCM reactor with methane under conditions at which oxygen atoms will readily migrate to the surface of the catalyst and activate the methane, while limiting the amount of free oxygen available to combust the desired products into CO and CO₂. The metal oxide catalysts may release one or more oxygen atoms before being recovered and regenerated with air in a separate reactor vessel, prior to being reintroduced into the OCM reactor. The above describe method to provide oxygen to the oxidative coupling of methane reaction is also referred to a chemical looping and may result in improved C2+ hydrocarbon yield and selectivity.

In step (a) of the process of the present invention, the methane:oxygen molar ratio in a reactor feed may be in the range of from 2:1 to 10:1, more preferably 3:1 to 6:1. In a case wherein in the present invention air is used as the oxidant in step (a), such methane:oxygen molar ratios correspond to methane:air molar ratios of 2:4.8 to 10:4.8 and 3:4.8 to 6:4.8, respectively.

Methane may be present in a reactor feed in a concentration of at least 35 mole%, more preferably at least 40 mole%, relative to the reactor feed. Further, methane may be present in a reactor feed in a concentration of at most 90 mole%, more preferably at most 85 mole%, most preferably at most 80 mole%, relative to the reactor feed. Thus, in the present invention, methane may for example be present in a reactor feed in a concentration in the range of from 35 to 90 mole%, more preferably 40 to 85 mole%, most preferably 40 to 80 mole%, relative to the reactor feed. In the context of the present invention, the components of said reactor feed are to be selected in an overall amount not to exceed 100 vol.%.

In general, the oxygen concentration in a reactor feed should be less than the concentration of oxygen that would form a flammable mixture at either the reactor inlet or the reactor outlet at the prevailing operating conditions.

The ratio of the methane to oxygen and volume percentages for the various components in a reactor feed are the ratio and volume percentages, respectively, at the entrance of the catalyst bed. Obviously, after entering the catalyst bed, at least a part of the oxygen and methane from the gas stream gets consumed.

In step (a), a reactor feed comprising methane and oxygen may be contacted with a methane oxidative coupling (OCM) catalyst so that methane is converted to one or more C2+ hydrocarbons, including ethylene. Suitably, the reactor temperature in said reaction step is in the range of from 500 to 1000 °C. Preferably, said conversion is effected at a reactor temperature in the range of from 700 to 1100 °C, more preferably 700 to 1000 °C, even more preferably 750 to 950 °C.

In a preferred embodiment, said conversion of methane to one or more C2+ hydrocarbons is effected at a reactor pressure in the range of from 0.1 to 20 bar, more preferably 0.5 to 20 bar, more preferably 1 to 15 bar, more preferably 2 to 10 bar.

According to the present invention, the above-mentioned methane oxidative coupling catalyst may be any methane oxidative coupling catalyst. Generally, the catalyst may contain one or more of manganese, one or more alkali metals (e.g. sodium) and tungsten. Preferably, the catalyst contains manganese, one or more alkali metals (e.g. sodium) and tungsten. Said carrier may be unsupported or supported. In particular, the catalyst may be a mixed metal oxide catalyst containing manganese, one or more alkali metals (e.g. sodium) and tungsten. Further, the catalyst may be a supported catalyst, such as a catalyst comprising manganese, one or more alkali metals (e.g. sodium) and tungsten on a carrier. The carrier may be any carrier, such as silica or a metal-containing carrier. A particular suitable catalyst comprises manganese, tungsten and sodium on a silica carrier (Mn-Na₂WO₄/SiO₂).

Suitable methane oxidative coupling catalysts are described in the following publications.

Chua et al. studied the oxidative coupling of methane for the production of ethylene over sodium-tungsten-manganese-supported silica catalyst (Na-W-Mn/SiO2) in Applied Catalysis A: General 343 (2008) 142-148.

The performance of Mn-Na₂WO₄/SiO₂ catalyst was further reviewed by Arndt et al. in Applied Catalysis A: General 425-426 (2012) 53-61 and Lee et al. in Fuel 106 (2013) 851-857.

US20130023709 describes the high throughput screening of catalyst libraries for the oxidative coupling of methane and tests various catalysts including catalysts comprising sodium, manganese and tungsten on silica and zirconia carriers.

US20140080699 describes a specific method for the preparation of catalysts such as Mn-Na₂WO₄/SiO₂ catalyst which is said to provide an improved catalyst material.

Various manganese and titanium-containing catalysts for the oxidative coupling of methane are researched in the literature and are disclosed in various patent publications including Gong et al. Catalysis Today 24 (1995), 259-261, Gong et al. Catalysis Today 24 (1995), 263-264, Jeon et al. Applied Catalysis A: General 464-465 (2013) 68-77, US4769508 and US20130178680.

The amount of the catalyst in said process is not essential. Preferably, a catalytically effective amount of the catalyst is used, that is to say an amount sufficient to promote a methane oxidative coupling reaction in step (a).

In step (a) of the process of the present invention, the gas hourly space velocity (GHSV; in m³ gas/m³ catalyst/hr) may typically be of from 100 to 50,000 hr⁻¹. Said GHSV is measured at standard temperature and pressure, namely 32 °F (0 °C) and 1 bara (100 kPa). In a preferred embodiment of the present invention, said GHSV is of from 2,500 to 25,000 hr⁻¹, more preferably of from 5,000 to 20,000 hr⁻¹, most preferably of from 7,500 to 15,000 hr⁻¹.

The catalyst used in step (a) may be a particulate catalyst, preferably a heterogeneous catalyst in the form of particles. The particles may be of any size suitable to be used in the reactor. The particles may be small enough to be used in a fluidized bed reactor. Alternatively, the particles may be arranged in a catalyst bed in the reactor. In that case the reactor may be a (multi-) tubular fixed bed reactor. Such a catalyst bed may comprise pellets, extrudates, or catalyst on a metal support (like a metal wire or metal flake). In addition to catalyst particles, the catalyst bed may also contain inert particles, i.e. catalytically inactive particles.

During step (a), ethane, ethylene and water are formed by oxidative coupling of methane. Further, carbon dioxide is formed as a by-product. During step (a) gas is fed to the reactor and an effluent is withdrawn from the reactor. The reactor effluent comprises ethylene, ethane, methane, carbon dioxide and water. Said methane comprises unconverted methane.

### Step (b)

In step (b) of the present integrated process, at least a part of the reactor effluent as obtained in step (a) is cooled to obtain a liquid stream comprising water and a gas stream comprising ethylene, ethane, methane and carbon dioxide. This may also be referred to as quenching which may be performed in a quencher.

In step (b) the reactor effluent may be cooled from the reaction temperature to a lower temperature, for example room temperature, so that the water condenses and can then be removed from the gas stream (reactor effluent).

In step (b), by cooling the reactor effluent, a liquid stream comprising water and a gas stream comprising ethylene, ethane, methane and carbon dioxide are obtained.

### Step (c)

In step (c) of the present integrated process, carbon dioxide is removed from at least a part of the gas stream comprising ethylene, ethane, methane and carbon dioxide as obtained in step (b) resulting in a gas stream comprising ethylene, ethane and methane.

Step (c) is preferably performed using one or more amines and/or by means of caustic treating. Caustic treating may be performed, for example, using a sodium hydroxide solution. A suitable carbon dioxide removal agent may be an aqueous solution of a base, for example sodium hydroxide or an amine.

### Step (d)

In step (d) of the present integrated process, a stream comprising methane, a stream comprising ethane and a stream comprising ethylene are recovered from at least a part of the gas stream comprising ethylene, ethane and methane as obtained in step (c).

These 3 streams may be recovered in step (d) by any separation method known to the skilled person, for example by means of distillation, absorption or adsorption or any combination thereof.

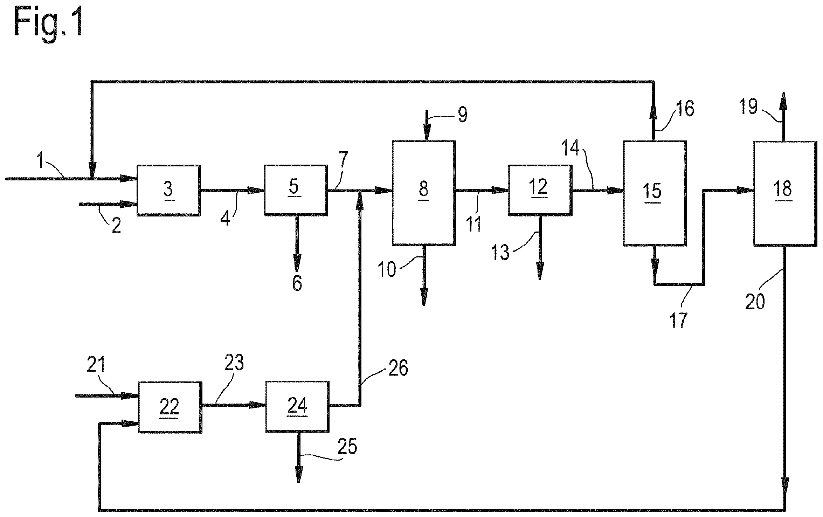

In one embodiment of step (d) (hereinafter "1^{st} embodiment"), at least a part of the gas stream comprising ethylene, ethane and methane as obtained in step (c) is separated into a stream comprising methane and a stream comprising ethylene and ethane. Said stream comprising ethylene and ethane is then further separated into a stream comprising ethylene and a stream comprising ethane. This embodiment of step (e) is illustrated in Figures 1-3, as further discussed below.

In another embodiment of step (d) (hereinafter "2^{nd} embodiment"), at least a part of the gas stream comprising ethylene, ethane and methane as obtained in step (c) is separated into a stream comprising methane and ethylene and a stream comprising ethane. Said stream comprising methane and ethylene is then further separated into a stream comprising methane and a stream comprising ethylene.

Generally, the above-mentioned 1^{st} embodiment is preferred. However, the above-mentioned 2^{nd} embodiment may be suitable in a case wherein in the stream or the combination of streams as fed to step (d), the amount of methane is relatively low and/or the amount of ethane is relatively high. The amount of methane in said stream(s) may be relatively low in a case wherein the conversion of methane in step (a) is relatively high. The amount of ethane in said stream(s) may be relatively high in a case wherein, in addition to ethane from the stream comprising ethane as obtained in step (d), fresh ethane is also fed to step (f), and at least a part of the stream resulting from step (f) is processed in one of the steps wherein the reactor effluent resulting from step (a) is processed, for example in step (b), (c) or (d).

### Step (e)

In step (e) of the present integrated process, at least a part of the stream comprising methane as obtained in step (d) is recycled to step (a). This recycle helps in maximising the production of ethylene in the present integrated process, as discussed above.

### Step (f)

In step (f) of the present integrated process, ethane from the stream comprising ethane as obtained in step (d) is converted to ethylene by subjecting the ethane to oxidative dehydrogenation (ODH) conditions. This conversion also helps in maximising the production of ethylene in the present integrated process, as discussed above.

The product of ODH step (f) comprises the dehydrogenated equivalent of ethane, that is to say ethylene. Such dehydrogenated equivalent is initially formed in said ethane ODH process. However, in said same process, said dehydrogenated equivalent may be further oxidized under the same conditions into the corresponding carboxylic acid. In the case of ethane, the product of said ODH process comprises ethylene and optionally acetic acid.

In ethane ODH step (f), oxygen and ethane, said ethane being from the stream comprising ethane as obtained in step (d), may be contacted with an ODH catalyst, resulting in a reactor effluent comprising ethylene, ethane, carbon dioxide and water. Ethane and oxygen (O₂) may be fed to the reactor together or separately. That is to say, one or more feed streams, suitably gas streams, comprising one or more of said 2 components may be fed to the reactor. For example, one feed stream comprising oxygen and ethane may be fed to the reactor. Alternatively, two or more feed streams, suitably gas streams, may be fed to the reactor, which feed streams may form a combined stream inside the reactor. For example, one feed stream comprising oxygen and another feed stream comprising ethane may be fed to the reactor separately.

In ethane ODH step (f), ethane and oxygen are suitably fed to a reactor in the gas phase.

Preferably, in ethane ODH step (f), that is to say during contacting ethane with oxygen in the presence of an ODH catalyst, the temperature is of from 300 to 500 °C. More preferably, said temperature is of from 310 to 450 °C, more preferably of from 320 to 420 °C, most preferably of from 330 to 420 °C.

Still further, in ethane ODH step (f), that is to say during contacting ethane with oxygen in the presence of an ODH catalyst, typical pressures are 0.1-30 or 0.1-20 bara (i.e. "bar absolute"). Further, preferably, said pressure is of from 0.1 to 15 bara, more preferably of from 1 to 8 bara, most preferably of from 3 to 8 bara.

In addition to oxygen and ethane, an inert gas may also be fed to step (f). Said inert gas may be selected from the group consisting of the noble gases and nitrogen (N₂). Preferably, the inert gas is nitrogen or argon, more preferably nitrogen. Said oxygen is an oxidizing agent, thereby resulting in oxidative dehydrogenation of ethane. Said oxygen may originate from any source, such as for example air. Alternatively, oxygen may be provided through a metal oxide, preferably mixed metal oxide, catalyst, which catalyst acts as both a source of oxygen as well as the catalyst for the ethane oxydehydrogenation reaction. The metal oxide catalyst is introduced to ODH reactor with ethane under conditions at which oxygen atoms will readily migrate to the surface of the catalyst and activate the ethane, while limiting the amount of free oxygen available to combust the desired products into CO and CO₂. The metal oxide catalysts may release one or more oxygen atoms before being recovered and regenerated with air in a separate reactor vessel, prior to being reintroduced into the ODH reactor. The above describe method to provide oxygen to the ethane oxydehydrogenation reaction is also referred to a chemical looping and may result in improved ethylene yield and selectivity.

Ranges for the molar ratio of oxygen to ethane which are suitable, are of from 0.01 to 1, more suitably 0.05 to 0.5. Said ratio of oxygen to ethane is the ratio before oxygen and ethane are contacted with the ODH catalyst. In other words, said ratio of oxygen to ethane is the ratio of oxygen as fed to ethane as fed. Obviously, after contact with the catalyst, at least a part of the oxygen and ethane gets consumed.

In step (f), the ODH catalyst may be a catalyst comprising a mixed metal oxide. Preferably, the ODH catalyst is a heterogeneous catalyst. Further, preferably, the ODH catalyst is a mixed metal oxide catalyst containing molybdenum, vanadium, niobium and optionally tellurium as the metals, which catalyst may have the following formula:

Mo₁VₐTe_{b}Nb_{c}Oₙ

wherein:
a, b, c and n represent the ratio of the molar amount of the element in question to the molar amount of molybdenum (Mo);
a (for V) is from 0.01 to 1, preferably 0.05 to 0.60, more preferably 0.10 to 0.40, more preferably 0.20 to 0.35, most preferably 0.25 to 0.30;
b (for Te) is 0 or from >0 to 1, preferably 0.01 to 0.40, more preferably 0.05 to 0.30, more preferably 0.05 to 0.20, most preferably 0.09 to 0.15;
c (for Nb) is from >0 to 1, preferably 0.01 to 0.40, more preferably 0.05 to 0.30, more preferably 0.10 to 0.25, most preferably 0.14 to 0.20; and
n (for 0) is a number which is determined by the valency and frequency of elements other than oxygen.

The amount of the catalyst in ethane ODH step (f) is not essential. Preferably, a catalytically effective amount of the catalyst is used, that is to say an amount sufficient to promote the ethane oxydehydrogenation reaction.

The ODH reactor that may be used in ethane ODH step (f) may be any reactor, including fixed-bed and fluidized-bed reactors. Suitably, the reactor is a fixed-bed reactor.

Examples of oxydehydrogenation processes, including catalysts and process conditions, are for example disclosed in above-mentioned US7091377, WO2003064035, US20040147393, WO2010096909 and US20100256432, the disclosures of which are herein incorporated by reference.

In ethane ODH step (f), water is formed which ends up in the product stream in addition to the desired ethylene product. Further, said product stream comprises unconverted ethane and carbon dioxide. That is to say, ethane ODH step (f) may result in a reactor effluent comprising ethylene, ethane, carbon dioxide and water.

At least a part of the reactor effluent comprising ethylene, ethane, carbon dioxide and water that may be obtained in step (f) may be fed to the above-mentioned step (b) wherein water is removed. Further, in the latter case, at least a part of the reactor effluent as obtained in step (a) may be fed to step (f). In said cases, both OCM effluent and ODH effluent are subjected together to the same water removal step (b).

### Step (g)

In optional step (g) of the present integrated process, at least a part of the reactor effluent that may be obtained in step (f) is cooled to obtain a liquid stream comprising water and a gas stream comprising ethylene, ethane and carbon dioxide. Steps (b) and (g) are not the same. This may also be referred to as quenching which may be performed in a quencher.

In step (g) the reactor effluent may be cooled from the reaction temperature to a lower temperature, for example room temperature, so that the water condenses and can then be removed from the gas stream (reactor effluent).

In step (g), by cooling the reactor effluent, a liquid stream comprising water and a gas stream comprising ethylene, ethane and carbon dioxide are obtained. In a case wherein the stream as fed to step (g) additionally comprises acetic acid, said acetic acid may be removed in step (g) together with the water from said stream, suitably together with the water as condensed from said stream. During or after step (g), additional water may be added to facilitate the removal of any acetic acid.

At least a part of the gas stream comprising ethylene, ethane and carbon dioxide as obtained in step (g) may be fed to the above-mentioned step (c) wherein carbon dioxide is removed. This is illustrated in Figure 1, as further discussed below.

### Step (h)

In optional step (h) of the present integrated process, carbon dioxide is removed from at least a part of the gas stream comprising ethylene, ethane and carbon dioxide as obtained in step (g) resulting in a gas stream comprising ethylene and ethane. Steps (c) and (h) are not the same.

Step (h) is preferably performed using one or more amines and/or by means of caustic treating. Caustic treating may be performed, for example, using a sodium hydroxide solution. A suitable carbon dioxide removal agent may be an aqueous solution of a base, for example sodium hydroxide or an amine.

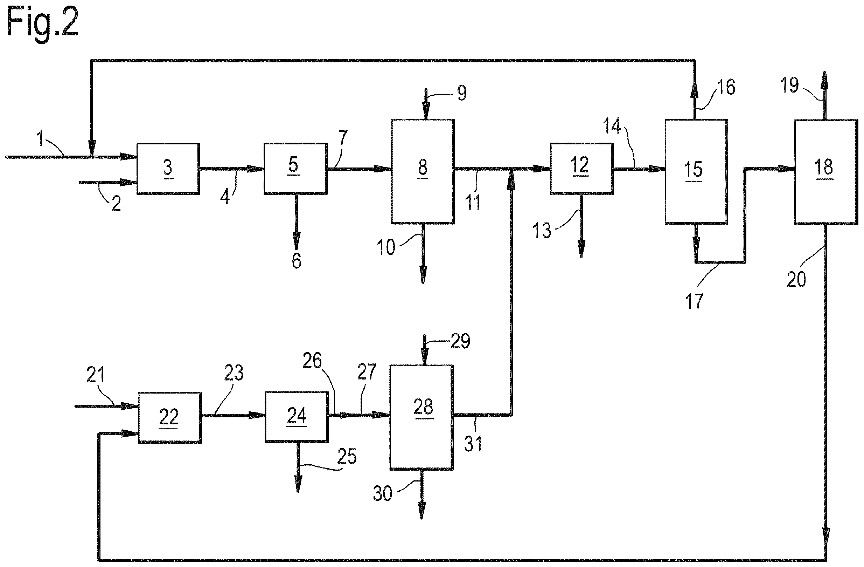

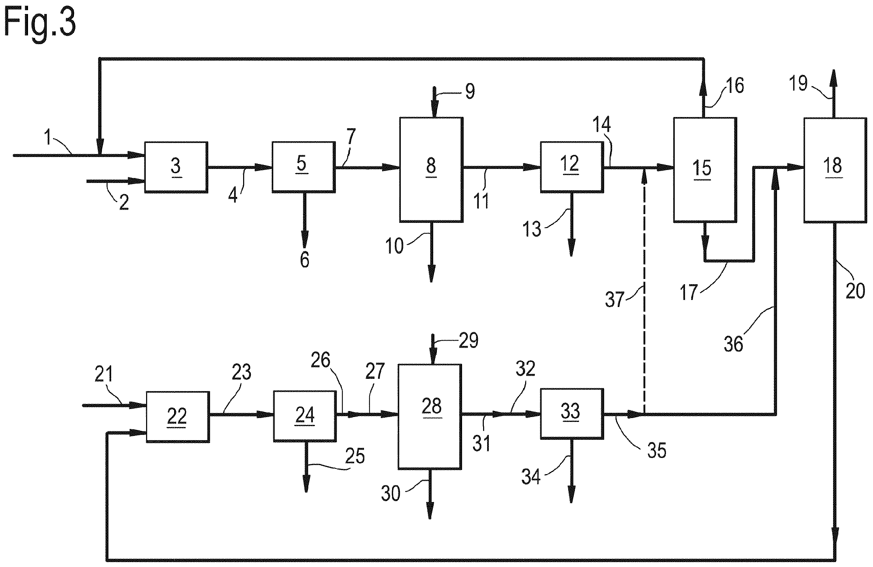

At least a part of the gas stream comprising ethylene and ethane as obtained in step (h) may be fed to the above-mentioned step (d) wherein a stream comprising methane, a stream comprising ethane and a stream comprising ethylene are recovered. This is illustrated in Figures 2 and 3, as further discussed below.

In the above-mentioned 1^{st} embodiment of step (d), at least a part of the gas stream comprising ethylene, ethane and methane as obtained in step (c) is separated into a stream comprising methane and a stream comprising ethylene and ethane, which latter stream comprising ethylene and ethane is then further separated into a stream comprising ethylene and a stream comprising ethane. It is preferred in said 1^{st} embodiment of step (d) that at least a part of the gas stream comprising ethylene and ethane as obtained in step (h) is fed to said substep of step (d) wherein the separated stream comprising ethylene and ethane is further separated into a stream comprising ethylene and a stream comprising ethane. This is illustrated in Figure 3, as further discussed below. Further, in said 1^{st} embodiment of step (d), at least a part of the gas stream comprising ethylene and ethane as obtained in step (h) may be fed to said substep of step (d) wherein at least a part of the gas stream comprising ethylene, ethane and methane as obtained in step (c) is separated into a stream comprising methane and a stream comprising ethylene and ethane. This is also illustrated in Figure 3, as further discussed below. An example wherein this is suitable, is a case wherein the gas stream comprising ethylene and ethane as obtained in step (h) additionally comprises carbon monoxide and/or methane and/or H₂ and/or inert gas, like N₂.

In the above-mentioned 2^{nd} embodiment of step (d), at least a part of the gas stream comprising ethylene, ethane and methane as obtained in step (c) is separated into a stream comprising ethane and a stream comprising methane and ethylene, which latter stream comprising methane and ethylene is then further separated into a stream comprising methane and a stream comprising ethylene. It is preferred in said 2^{nd} embodiment of step (d) that at least a part of the gas stream comprising ethylene and ethane as obtained in step (h) is fed to said substep of step (d) wherein at least a part of the gas stream comprising ethylene, ethane and methane as obtained in step (c) is separated into a stream comprising ethane and a stream comprising methane and ethylene.

### Optional drying step after step (c)

In an optional drying step between steps (c) and (d), water may be removed from the gas stream comprising ethylene, ethane and methane as obtained in step (c) which latter stream may additionally comprise water, resulting in a gas stream comprising ethylene, ethane and methane. Said water may originate from the carbon dioxide removal agent used in step (c) .

In the case of such optional drying step between steps (c) and (d), at least a part of the gas stream comprising ethylene and ethane as obtained in step (h) may be fed to said drying step. This is illustrated in Figure 2, as further discussed below. The gas stream comprising ethylene and ethane as obtained in step (h) may additionally comprise water. Said water may originate from the carbon dioxide removal agent used in step (h) .

### Optional drying step after step (h)

In an optional drying step after step (h), water may be removed from the gas stream comprising ethylene and ethane as obtained in step (h) which latter stream may additionally comprise water, resulting in a gas stream comprising ethylene and ethane. Said water may originate from the carbon dioxide removal agent used in step (h). Said optional drying step after step (h) and the above-mentioned optional drying step between steps (c) and (d) are not the same.

In the case of such optional drying step after step (h) and the above-mentioned 1^{st} embodiment of step (d), it is preferred that at least a part of the gas stream comprising ethylene and ethane as obtained in said drying step is fed to the substep of step (d) wherein the separated stream comprising ethylene and ethane is further separated into a stream comprising ethylene and a stream comprising ethane. This is illustrated in Figure 3, as further discussed below. Further, in the case of such optional drying step after step (h) and the above-mentioned 1^{st} embodiment of step (d), at least a part of the gas stream comprising ethylene and ethane as obtained in said drying step may be fed to said substep of step (d) wherein a stream comprising methane and a stream comprising ethylene and ethane are produced. This is also illustrated in Figure 3, as further discussed below. An example wherein this is suitable, is a case wherein the gas stream comprising ethylene and ethane as obtained in step (h) additionally comprises carbon monoxide and/or methane and/or H₂ and/or inert gas, like N₂.

Further, in the case of such optional drying step after step (h) and the above-mentioned 2^{nd} embodiment of step (d), it is preferred that at least a part of the gas stream comprising ethylene and ethane as obtained in said drying step is fed to said substep of step (d) wherein a stream comprising ethane and a stream comprising methane and ethylene are produced.

### Figures

The process of the present invention is further illustrated by Figures 1-3.

In Figure 1, an oxidative coupling of methane (OCM) configuration is shown. Said OCM configuration comprises OCM unit 3, water condensation unit 5, carbon dioxide removal unit 8, drying unit 12 and separation units 15 and 18. Said separation units 15 and 18 are distillation columns. Further, in Figure 1, an oxidative dehydrogenation (ODH) configuration integrated with said OCM configuration is also shown. Said ODH configuration comprises ODH unit 22 and water condensation unit 24.

In said Figure 1, stream 1 comprising methane and stream 2 comprising an oxidizing agent are fed to OCM unit 3 operating under OCM conditions. Product stream 4 coming from OCM unit 3 comprises ethane, ethylene, methane, carbon dioxide and water. Said stream 4 is fed to water condensation unit 5. In water condensation unit 5, water is removed by condensation via stream 6. In Figure 1, stream 7 coming from water condensation unit 5, which comprises ethane, ethylene, methane and carbon dioxide, is fed to carbon dioxide removal unit 8.

Carbon dioxide removal agent is fed to carbon dioxide removal unit 8 via stream 9. Said carbon dioxide removal agent may be an aqueous solution of a base, for example sodium hydroxide or an amine. Carbon dioxide removal unit 8 may comprise a subunit wherein carbon dioxide is removed by an aqueous solution of an amine and a downstream subunit wherein carbon dioxide is removed by an aqueous solution of sodium hydroxide. Carbon dioxide is removed via aqueous stream 10. Stream 11 coming from carbon dioxide removal unit 8, which comprises ethane, ethylene, methane and water, is fed to drying unit 12. In drying unit 12, water is removed via stream 13. Stream 14 coming from drying unit 12, which comprises ethane, ethylene and methane is fed to separation unit 15.

In separation unit 15, stream 14 comprising ethane, ethylene and methane is separated into a top stream 16 comprising methane and a bottom stream 17 comprising ethane and ethylene. Stream 17 is fed to separation unit 18. In separation unit 18, stream 17 is separated into a top stream 19 comprising ethylene and a bottom stream 20 comprising ethane.

Stream 16 comprising methane is recycled to OCM unit 3.

Further, in said Figure 1, stream 20 comprising ethane and stream 21 comprising an oxidizing agent are fed to ODH unit 22 containing an ODH catalyst and operating under ODH conditions. The source of oxygen as fed to OCM unit 3 and ODH unit 22 may be the same. For example, the same oxygen generation unit (not shown in Figure 1) may be used for generating streams 2 and 21. Product stream 23 coming from ODH unit 22 comprises water, ethane, ethylene, carbon dioxide and any acetic acid. Said stream 23 is fed to water condensation unit 24. In water condensation unit 24, water and any acetic acid are removed by condensation via stream 25.

In Figure 1, stream 26 coming from water condensation unit 24, which comprises ethane, ethylene and carbon dioxide, is fed to carbon dioxide removal unit 8 which is part of the OCM configuration.

In Figure 2, an OCM configuration and an ODH configuration integrated with said OCM configuration are shown. The OCM configuration is identical to that of Figure 1. In Figure 2, the ODH configuration comprises ODH unit 22, water condensation unit 24 and carbon dioxide removal unit 28.

The process of Figure 2 is the same as the process of Figure 1, with the exception that stream 26 coming from water condensation unit 24 which is part of the ODH configuration, which stream 35 comprises ethane, ethylene and carbon dioxide, is not fed to carbon dioxide removal unit 8 which is part of the OCM configuration, but is fed, via stream 27, to carbon dioxide removal unit 28 which is part of the ODH configuration. Carbon dioxide removal agent is fed to carbon dioxide removal unit 28 via stream 29. Said carbon dioxide removal agent may be an aqueous solution of a base, for example sodium hydroxide or an amine. Carbon dioxide removal unit 28 may comprise a subunit wherein carbon dioxide is removed by an aqueous solution of an amine and a downstream subunit wherein carbon dioxide is removed by an aqueous solution of sodium hydroxide. Carbon dioxide is removed via aqueous stream 30. In Figure 2, stream 31 coming from carbon dioxide removal unit 28, which comprises ethane, ethylene and water, is fed to drying unit 12 which is part of the OCM configuration.

In Figure 3, an OCM configuration and an ODH configuration integrated with said OCM configuration are shown. The OCM configuration is identical to that of Figures 1 and 2. In Figure 3, the ODH configuration comprises ODH unit 22, water condensation unit 24, carbon dioxide removal unit 28 and drying unit 33.

The process of Figure 3 is the same as the process of Figure 2, with the exception that stream 31 coming from carbon dioxide removal unit 28 which is part of the ODH configuration, which stream 31 comprises ethane, ethylene and water, is not fed to drying unit 12 which is part of the OCM configuration, but is fed, via stream 32, to drying unit 33 which is part of the ODH configuration. In drying unit 33, water is removed via stream 34. Stream 35 coming from drying unit 33 comprises ethane and ethylene. Said stream 35 is fed to separation unit 18 which is part of the OCM configuration. Alternatively, for example in a case wherein said stream 35 additionally comprises carbon monoxide and/or methane and/or H₂ and/or inert gas, like N₂, said stream 35 may be fed, via stream 37, to separation unit 15 which is part of the OCM configuration.

In Figures 1-3, the integration of OCM and ODH configurations may comprise one or more compressors (not shown in Figures 1-3), as exemplified below.

In Figure 1, the integration of OCM and ODH configurations may comprise 1 compressor. Said compressor may be placed between carbon dioxide removal unit 8 and the point at which streams 7 and 26 are combined.

Further, in Figure 1, the integration of OCM and ODH configurations may comprise 2 compressors. A first compressor may be placed between carbon dioxide removal unit 8 and the point at which streams 7 and 26 are combined; and a second compressor may be placed between carbon dioxide removal unit 8 and drying unit 12. Alternatively, a first compressor may be placed between water condensation unit 5 and the point at which streams 7 and 26 are combined; and a second compressor may be placed between water condensation unit 24 and the point at which streams 7 and 26 are combined.

Still further, in Figure 1, the integration of OCM and ODH configurations may comprise 3 compressors. A first compressor may be placed between carbon dioxide removal unit 8 and drying unit 12; a second compressor may be placed between water condensation unit 5 and the point at which streams 7 and 26 are combined; and a third compressor may be placed between water condensation unit 24 and the point at which streams 7 and 26 are combined.

In Figures 2 and 3, the integration of OCM and ODH configurations may comprise 2 compressors. A first compressor may be placed between water condensation unit 5 and carbon dioxide removal unit 8; and a second compressor may be placed between water condensation unit 24 and carbon dioxide removal unit 28.

Further, in Figure 2, the integration of OCM and ODH configurations may comprise 3 compressors. A first compressor may be placed between water condensation unit 5 and carbon dioxide removal unit 8; a second compressor may be placed between water condensation unit 24 and carbon dioxide removal unit 28; and a third compressor may be placed between drying unit 12 and the point at which streams 11 and 31 are combined.

## Claims

1. A process for oxidative coupling of methane (OCM), comprising the steps of:
(a) contacting, in a reactor, oxygen and methane with an OCM catalyst, resulting in a reactor effluent comprising ethylene, ethane, methane, carbon dioxide and water;
(b) cooling at least a part of the reactor effluent as obtained in step (a) to obtain a liquid stream comprising water and a gas stream comprising ethylene, ethane, methane and carbon dioxide;
(c) removing carbon dioxide from at least a part of the gas stream comprising ethylene, ethane, methane and carbon dioxide as obtained in step (b) resulting in a gas stream comprising ethylene, ethane and methane;
(d) recovering a stream comprising methane, a stream comprising ethane and a stream comprising ethylene from at least a part of the gas stream comprising ethylene, ethane and methane as obtained in step (c);
(e) recycling at least a part of the stream comprising methane as obtained in step (d) to step (a);
(f) converting ethane from the stream comprising ethane as obtained in step (d) to ethylene by subjecting the ethane to oxidative dehydrogenation (ODH) conditions.

2. Process according to claim 1, wherein step (f) comprises contacting, in a reactor, oxygen and ethane from the stream comprising ethane as obtained in step (d) with an ODH catalyst, resulting in a reactor effluent comprising ethylene, ethane, carbon dioxide and water, and said process additionally comprises the step of:
(g) cooling at least a part of the reactor effluent as obtained in step (f) to obtain a liquid stream comprising water and a gas stream comprising ethylene, ethane and carbon dioxide.

3. Process according to claim 2, wherein at least a part of the gas stream comprising ethylene, ethane and carbon dioxide as obtained in step (g) is fed to step (c).

4. Process according to claim 2, which additionally comprises the step of:
(h) removing carbon dioxide from at least a part of the gas stream comprising ethylene, ethane and carbon dioxide as obtained in step (g) resulting in a gas stream comprising ethylene and ethane.

5. Process according to claim 4, wherein at least a part of the gas stream comprising ethylene and ethane as obtained in step (h) is fed to step (d).

6. Process according to claim 4, wherein step (d) comprises separating at least a part of the gas stream comprising ethylene, ethane and methane as obtained in step (c) into a stream comprising methane and a stream comprising ethylene and ethane, and further separating the latter stream comprising ethylene and ethane into a stream comprising ethylene and a stream comprising ethane, and wherein at least a part of the gas stream comprising ethylene and ethane as obtained in step (h) is fed to said substep of step (d) wherein the separated stream comprising ethylene and ethane is further separated into a stream comprising ethylene and a stream comprising ethane.

7. Process according to claim 4, wherein in a drying step between steps (c) and (d), water is removed from the gas stream comprising ethylene, ethane and methane as obtained in step (c) which latter stream additionally comprises water, resulting in a gas stream comprising ethylene, ethane and methane, and wherein at least a part of the gas stream comprising ethylene and ethane as obtained in step (h) which latter stream additionally comprises water, is fed to said drying step.

8. Process according to claim 4, wherein step (d) comprises separating at least a part of the gas stream comprising ethylene, ethane and methane as obtained in step (c) into a stream comprising methane and a stream comprising ethylene and ethane, and further separating the latter stream comprising ethylene and ethane into a stream comprising ethylene and a stream comprising ethane, wherein in a drying step after step (h), water is removed from the gas stream comprising ethylene and ethane as obtained in step (h) which latter stream additionally comprises water, resulting in a gas stream comprising ethylene and ethane, and wherein at least a part of the gas stream comprising ethylene and ethane as obtained in said drying step is fed to said substep of step (d) wherein the separated stream comprising ethylene and ethane is further separated into a stream comprising ethylene and a stream comprising ethane.

9. Process according to claim 1, wherein step (f) comprises contacting, in a reactor, oxygen and ethane from the stream comprising ethane as obtained in step (d) with an ODH catalyst, resulting in a reactor effluent comprising ethylene, ethane, carbon dioxide and water, and wherein at least a part of the reactor effluent comprising ethylene, ethane, carbon dioxide and water as obtained in step (f) is fed to step (b).

## Patentansprüche

1. Vorgang zum oxidativen Koppeln von Methan (*oxidative coupling of methane* - OCM), der die folgenden Schritte umfasst:
(a) Inberührungbringen von Sauerstoff und Methan mit einem OCM-Katalysator in einem Reaktor, das einen Reaktorausfluss ergibt, der Ethylen, Ethan, Methan, Kohlendioxid und Wasser umfasst;
(b) Kühlen wenigstens eines Teils des wie in Schritt (a) gewonnenen Reaktorausflusses, um einen Flüssigkeitsstrom, der Wasser umfasst, und einen Gasstrom, der Ethylen, Ethan, Methan und Kohlendioxid umfasst, zu gewinnen;
(c) Entfernen von Kohlendioxid aus wenigstens einem Teil des wie in Schritt (b) gewonnenen Gasstroms, der Ethylen, Ethan, Methan und Kohlendioxid umfasst, das einen Gasstrom ergibt, der Ethylen, Ethan und Methan umfasst;
(d) Zurückgewinnen eines Stroms, der Methan umfasst, eines Stroms, der Ethan umfasst, und eines Stroms, der Ethylen umfasst, aus wenigstens einem Teil des wie in Schritt (c) gewonnenen Gasstroms, der Ethylen, Ethan und Methan umfasst;
(e) Wiederverwerten wenigstens eines Teils des wie in Schritt (d) bis Schritt (a) gewonnenen Stroms, der Methan umfasst;
(f) Umwandeln von Ethan aus dem wie in Schritt (d) gewonnenen Strom,, der Ethan umfasst, in Ethylen, indem das Ethan Bedingungen der oxidativen Dehydrierung (*oxidative dehydrogenation* - ODH) ausgesetzt wird.

2. Vorgang nach Anspruch 1, wobei Schritt (f) das Inberührungbringen von Sauerstoff und Ethan aus dem wie in Schritt (d) gewonnenen Strom, der Ethan umfasst, mit einem ODH-Katalysator in einem Reaktor umfasst, das einen Reaktorausfluss ergibt, der Ethylen, Ethan, Kohlendioxid und Wasser umfasst, und der Vorgang zusätzlich den folgenden Schritt umfasst:
(g) Kühlen wenigstens eines Teils des wie in Schritt (f) gewonnenen Reaktorausflusses, um einen Flüssigkeitsstrom, der Wasser umfasst, und einen Gasstrom, der Ethylen, Ethan und Kohlendioxid umfasst, zu gewinnen.

3. Vorgang nach Anspruch 2, wobei wenigstens ein Teil des wie in Schritt (g) gewonnenen Gasstroms, der Ethylen, Ethan und Kohlendioxid umfasst, Schritt (c) zugeführt wird.

4. Vorgang nach Anspruch 2, der zusätzlich den folgenden Schritt umfasst:
(h) Entfernen von Kohlendioxid aus wenigstens einem Teil des wie in Schritt (g) gewonnenen Gasstroms, der Ethylen, Ethan und Kohlendioxid umfasst, das einen Gasstrom ergibt, der Ethylen und Ethan umfasst.

5. Vorgang nach Anspruch 4, wobei wenigstens ein Teil des wie in Schritt (h) gewonnenen Gasstroms, der Ethylen und Ethan umfasst, Schritt (d) zugeführt wird.

6. Vorgang nach Anspruch 4, wobei Schritt (d) ein Trennen wenigstens eines Teils des wie in Schritt (c) gewonnenen Gasstroms, der Ethylen, Ethan und Methan umfasst, in einen Strom, der Methan umfasst, und einen Strom, der Ethylen und Ethan umfasst, umfasst, und ferner das Trennen des letzteren Stroms, der Ethylen und Ethan umfasst, in einen Strom, der Ethylen umfasst, und einen Strom, der Ethan umfasst, umfasst, und wobei wenigstens ein Teil des wie in Schritt (h) gewonnenen Gasstroms, der Ethylen und Ethan umfasst, dem Unterschritt von Schritt (d) zugeführt wird, wobei der getrennte Strom, der Ethylen und Ethan umfasst, ferner in einen Strom, der Ethylen umfasst, und einen Strom, der Ethan umfasst, getrennt wird.

7. Vorgang nach Anspruch 4, wobei in einem Trocknungsschritt zwischen den Schritten (c) und (d) Wasser aus dem wie in Schritt (c) gewonnenen Gasstrom, der Ethylen, Ethan und Methan umfasst, entfernt wird, wobei letzterer Strom zusätzlich Wasser umfasst, der einen Gasstrom ergibt, der Ethylen, Ethan und Methan umfasst, und wobei wenigstens ein Teil des wie in Schritt (h) gewonnenen Gasstroms, der Ethylen und Ethan umfasst, wobei letzterer Strom zusätzlich Wasser umfasst, dem Trocknungsschritt zugeführt wird.

8. Vorgang nach Anspruch 4, wobei Schritt (d) das Trennen wenigstens eines Teils des wie in Schritt (c) gewonnenen Gasstroms, der Ethylen, Ethan und Methan umfasst, in einen Strom, der Methan umfasst, und einen Strom, der Ethylen und Ethan umfasst, umfasst, und ferner das Trennen des letzteren Stroms, der Ethylen und Ethan umfasst, in einen Strom, der Ethylen umfasst, und einen Strom, der Ethan umfasst, umfasst, wobei in einem Trocknungsschritt nach Schritt (h) Wasser aus dem wie in Schritt (h) gewonnenen Gasstrom, der Ethylen und Ethan umfasst, entfernt wird, wobei letzterer Strom zusätzlich Wasser umfasst, das einen Gasstrom ergibt, der Ethylen und Ethan umfasst, und wobei wenigstens ein Teil des wie in dem Trocknungsschritt gewonnenen Gasstroms, der Ethylen und Ethan umfasst, dem Unterschritt von Schritt (d) zugeführt wird, wobei der getrennte Strom, der Ethylen und Ethan umfasst, ferner in einen Strom, der Ethylen umfasst, und einen Strom, der Ethan umfasst, getrennt wird.

9. Vorgang nach Anspruch 1, wobei Schritt (f) das Inberührungbringen von Sauerstoff und Ethan aus dem wie in Schritt (d) gewonnenen Strom, der Ethan umfasst, mit einem ODH-Katalysator in einem Reaktor umfasst, das einen Reaktorausfluss ergibt, der Ethylen, Ethan, Kohlendioxid und Wasser umfasst, und wobei wenigstens ein Teil des wie in Schritt (f) gewonnenen Reaktorausflusses, der Ethylen, Ethan, Kohlendioxid und Wasser umfasst, Schritt (b) zugeführt wird.

## Revendications

1. Procédé de couplage oxydant du méthane (OCM), comprenant les étapes consistant à :
(a) mettre en contact, dans un réacteur, de l'oxygène et du méthane avec un catalyseur OCM, pour obtenir un effluent de réacteur comprenant de l'éthylène, de l'éthane, du méthane, du dioxyde de carbone et de l'eau ;
(b) refroidir au moins une partie de l'effluent du réacteur tel qu'obtenu à l'étape (a) pour obtenir un flux liquide comprenant de l'eau et un flux gazeux comprenant de l'éthylène, de l'éthane, du méthane et du dioxyde de carbone ;
(c) éliminer le dioxyde de carbone d'au moins une partie du flux gazeux comprenant de l'éthylène, de l'éthane, du méthane et du dioxyde de carbone tel qu'obtenu à l'étape (b), pour obtenir un flux gazeux comprenant de l'éthylène, de l'éthane et du méthane ;
(d) récupérer un flux comprenant du méthane, un flux comprenant de l'éthane et un flux comprenant de l'éthylène à partir d'au moins une partie du flux gazeux comprenant de l'éthylène, de l'éthane et du méthane tel qu'obtenu à l'étape (c) ;
(e) recycler au moins une partie du flux comprenant le méthane tel qu'obtenu de l'étape (d) à l'étape (a) ;
(f) convertir l'éthane du flux comprenant l'éthane obtenu à l'étape (d) en éthylène en soumettant l'éthane à des conditions de déshydrogénation oxydante (ODH).

2. Procédé selon la revendication 1, dans lequel l'étape (f) comprend la mise en contact, dans un réacteur, de l'oxygène et de l'éthane à partir du flux comprenant de l'éthane tel qu'obtenu à l'étape (d) avec un catalyseur ODH, pour obtenir un effluent de réacteur comprenant de l'éthylène, de l'éthane, du dioxyde de carbone et de l'eau, et ledit procédé comprend en outre l'étape consistant à :
(g) refroidir au moins une partie de l'effluent du réacteur tel qu'obtenu à l'étape (f) pour obtenir un flux liquide comprenant de l'eau et un flux gazeux comprenant de l'éthylène, de l'éthane et du dioxyde de carbone.

3. Procédé selon la revendication 2, dans lequel au moins une partie du flux gazeux comprenant de l'éthylène, de l'éthane et du dioxyde de carbone tel qu'obtenu à l'étape (g) est alimenté à l'étape (c).

4. Procédé selon la revendication 2, qui comprend en outre l'étape consistant à :
(h) éliminer le dioxyde de carbone d'au moins une partie du flux gazeux comprenant de l'éthylène, de l'éthane et du dioxyde de carbone tel qu'obtenu à l'étape (g), pour obtenir un flux gazeux comprenant de l'éthylène et de l'éthane.

5. Procédé selon la revendication 4, dans lequel au moins une partie du flux gazeux comprenant de l'éthylène et de l'éthane tel qu'obtenu à l'étape (h) est alimentée à l'étape (d).

6. Procédé selon la revendication 4, dans lequel l'étape (d) comprend la séparation d'au moins une partie du flux gazeux comprenant de l'éthylène, de l'éthane et du méthane tel qu'obtenu à l'étape (c) en un flux comprenant du méthane et un flux comprenant de l'éthylène et de l'éthane, et la séparation en outre de ce dernier flux comprenant de l'éthylène et de l'éthane en un flux comprenant de l'éthylène et un flux comprenant de l'éthane, et dans lequel au moins une partie du flux gazeux comprenant de l'éthylène et de l'éthane obtenu à l'étape (h) est alimentée à ladite sous-étape de l'étape (d), le flux séparé comprenant de l'éthylène et de l'éthane étant en outre séparé en un flux comprenant de l'éthylène et un flux comprenant de l'éthane.

7. Procédé selon la revendication 4, dans lequel, dans une étape de séchage entre les étapes (c) et (d), l'eau est éliminée du flux gazeux comprenant de l'éthylène, de l'éthane et du méthane tel qu'obtenu à l'étape (c), lequel dernier flux comprenant en outre de l'eau, pour obtenir un flux gazeux comprenant de l'éthylène, de l'éthane et du méthane, et au moins une partie du flux gazeux comprenant de l'éthylène et de l'éthane tel qu'obtenu à l'étape (h), lequel dernier flux comprenant en outre de l'eau, est alimentée à ladite étape de séchage.

8. Procédé selon la revendication 4, dans lequel l'étape (d) comprend la séparation d'au moins une partie du flux gazeux comprenant de l'éthylène, de l'éthane et du méthane tel qu'obtenu à l'étape (c) en un flux comprenant du méthane et un flux comprenant de l'éthylène et de l'éthane, et la séparation en outre de ce dernier flux comprenant de l'éthylène et de l'éthane en un flux comprenant de l'éthylène et un flux comprenant de l'éthane, dans lequel, dans une étape de séchage après l'étape (h), l'eau est éliminée du flux gazeux comprenant de l'éthylène et de l'éthane, obtenu à l'étape (h), lequel dernier flux comprend en outre de l'eau, pour obtenir un flux gazeux comprenant de l'éthylène et de l'éthane, et au moins une partie du flux gazeux comprenant de l'éthylène et de l'éthane tel qu'obtenu à ladite étape de séchage est alimentée à ladite sous-étape de l'étape (d), le flux séparé comprenant de l'éthylène et de l'éthane étant en outre séparé en un flux comprenant de l'éthylène et un flux comprenant de l'éthane.

9. Procédé selon la revendication 1, dans lequel l'étape (f) comprend la mise en contact, dans un réacteur, de l'oxygène et de l'éthane à partir du flux comprenant de l'éthane tel qu'obtenu à l'étape (d) avec un catalyseur ODH, pour obtenir un effluent de réacteur comprenant de l'éthylène, de l'éthane, du dioxyde de carbone et de l'eau, et au moins une partie de l'effluent du réacteur comprenant de l'éthylène, de l'éthane, du dioxyde de carbone et de l'eau tel qu'obtenu à l'étape (f) est alimentée à l'étape (b).
